(19) Europäisches Patentamt
European Patent Office
Office européen des brevets

(11) Publication number: 0 212 972 A2

# (12) EUROPEAN PATENT APPLICATION

(21) Application number: 86306408.5

(22) Date of filing: 19.08.86

(51) Int. Cl.⁴: C 12 P 13/22, C 12 N 1/20 // (C12N1/20, C12R1:15)

(30) Priority: 20.08.85 US 767364

(43) Date of publication of application: 04.03.87 Bulletin 87/10

(84) Designated Contracting States: BE DE FR GB IT NL SE

(71) Applicant: ALLELIX INC., 6850 Goreway Drive, Mississauga Ontario, L4V 1P1 (CA)

(72) Inventor: Misawa, Masa, 12 Downpatrick Crescent, Weston Ontario M9R 4A4 (CA)
Inventor: Evans, Christopher Thomas, 1 Jewel Cottage Howland Road, Marden Tonbridge Kent (GB)

(74) Representative: Goldin, Douglas Michael et al, J.A. KEMP & CO. 14, South Square Gray's Inn, London WC1R 5EU (GB)

## (54) Bioconversion process.

(57) Acetamidocinnamate is efficiently converted in high yield, to L-phenylalanine, using enzymes synthesized by microbial cells of certain isolated strains of the genera Alcaligenes, Pseudomonas, Corynebacterium, Brevibakterium, Bacillus and Micrococcus forms. The cells of these isolates synthesize enzymes capable of transforming acetamidocinnamate to L-phenylalanine, when grown in the presence of acetamidocinnamate. The enzymes are synthesized in cells grown on a wide range of carbon and nitrogen sources, in the presence of a suitable amount of acetamidocinnamate (ACA).

The enzymes catalysing this transformation are maximally synthesised in cells grown in medium containing, as carbon source, glucose, phenylalanine, lactate, pyruvate, citrate, sorbitol, acetamidocinnamate and as nitrogen source, phenylalanine, histidine, acetamidocinnamate. In a medium containing lactate, phenylalanine and acetamidocinnamate, the cells maximally synthesise a specific permease (translocase) system for transport of substrate into the cell; a deaminase (dehydrate) enzyme which converts ACA to acetate, $NH_4^+$ and phenylpyruvate; a phenylalanine dehydrogenase enzyme which reaminates phenylpyruvate to produce L-phenylalanine and a number of coupling dehydrogenases (pyruvate dh., lactate dh.) which regenerate and supply reduced cofactor, NADH, to drive the final phenylalanine-synthesising dehydrogenase reaction. Thus, cells or extracts of these microorganisms contain a naturally-coupled system of enzymes capable of transforming acetamidocinnamic acid, or related compounds, to specifically L-phenylalanine or other related amino acids or derivatives thereof.

EP 0 212 972 A2

# BIOCONVERSION PROCESS

## FIELD OF THIS INVENTION

THIS INVENTION relates to microbiological processes, a biochemical pathway, an interaction of enzyme-catalysed reactions and to appropriate microorganisms for use therein. More specifically, this invention relates to a novel process for the microbiological production of L-phenylalanine, to a novel sequence of enzymes synthesised by microorganisms and capable of producing amino acids or derivatives thereof and to a novel microbial isolate for use therein.

## INTRODUCTION AND BACKGROUND OF THE INVENTION

L-Phenylalanine of chemical formula

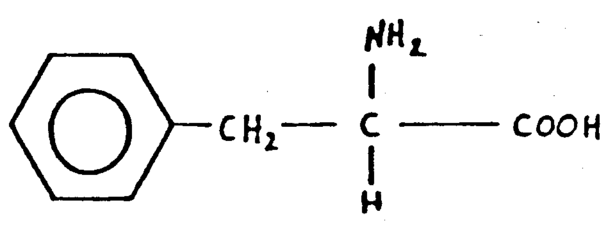

is an essential amino acid, an essential component in human nutrition and is of commercial importance as an intermediate in chemical synthesis in dietary foodstuffs, medical applications and, most significant of all, it is

used for production of NUTRASWEET (Aspartame : N,L-aspartyl-L-phenylalanine-1-methyl ester) a popular artificial sweetener by reaction with aspartic acid and methanol. The amino acid can be prepared by chemical synthesis, using benzaldehyde, glycine and acetic anhydride with an optical resolution step in the final stages, or by chemical synthesis or by fermentation processes from t-cinnamate or L-tyrosine. It has also been made by direct fermentation of a carbon source, such as molasses, glucose, methanol using various strains of Coryneform bacteria and genetically-manipulated strains of *E. coli*; the phenylalanine is recovered from the culture broth as the L-isomer. L-phenylalanine may also be isolated commercially from proteins such as ovalbumin, lactalbumin, zein and fibrin. This amino acid has also been prepared by various biotransformations such as using yeast phenylalanine ammonia lyase to transform trans-cinnamic acid to phenylalanine, the use of N-acetyl-D,L-phenylalanine and deacetylase (acylase) enzymes and the use of keto acid precursors and transamination reactions from a wide variety of microorganisms.

It is an object of the present invention to provide a novel process for production of L-phenylalanine.

It is an object of this present invention to provide a novel and improved process for the production of L-phenylalanine.

It is a further object of the present invention to provide a novel microbiological isolate useful in producing L-phenylalanine.

It is a further object of this invention to provide a novel and improved microbiological process for preparing an amino acid, more specifically L-phenylalanine, in accordance with a system of coupled enzymatic reactions.

It is the intention of the present invention to provide a process whereby the aforementioned system of coupled reactions is naturally synthesised by wild type microorganisms.

In addition it is the object of this present invention to provide a method for producing L-phenylalanine in accordance with a deaminase reaction yielding phenylpyruvic acid which is reaminated via a dehydrogenase reaction using reduced cofactors generated by other coupled dehydrogenase systems.

It is a further object of the present invention to increase phenylalanine production, in accordance with the deaminase and dehydrogenase systems, by fully inducing synthesis of a specific acetamidocinnamate permease, deaminase and phenylalanine dehydrogenase by manipulation of the growth mediums and reaction conditions.

It is also the object of this invention to maximise rates of phenylalanine synthesis by manipulating the supply of essential reduced cofactors required by

supplying cosubstrates capable of efficiently generating such reducing equivalents. Such substrates include formate, lactate, pyruvate, citrate, oxaloacetate, acetate, and glucose.

SUMMARY OF THE INVENTION

According to the present invention, L-phenylalanine is produced microbiologically from acetamidocinnamate (ACA), using novel microbiological isolate strains, or, more particularly, using enzymes synthesized by novel microbiological isolate strains. From the chemical viewpoint, the process may be represented thus:-

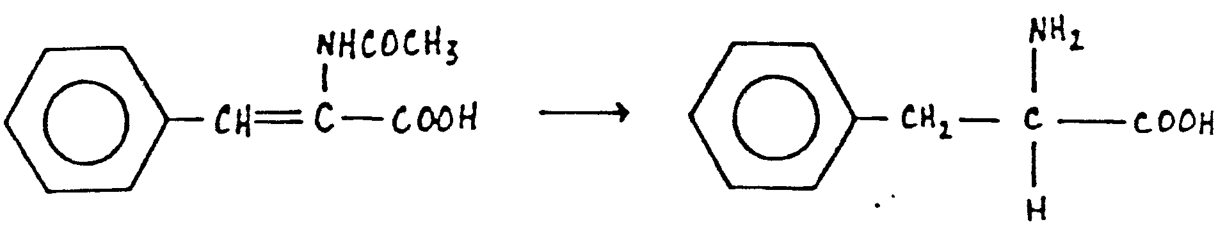

ACA L-Phe

The starting, substrate material ACA is commercially available, at reasonable prices.

The useful strains of microorganisms for use in the present invention may belong to one of several genera, such as Alcaligenes, Pseudomonas, Corynebacterium or

Brevibacterium Bacillus and Micrococcus. Each of the useful strains, when cultivated in an aqueous medium containing ACA, synthesizes at least one enzyme which participates in the chemical transformation of ACA to L-Phe in the said aqueous medium. The strains may be isolated from soil and sewage, using continuous enrichment methods generally familiar to those skilled in the art, and using enrichment media containing ACA as the sole carbon and nitrogen source.

The present invention also relates to a novel and improved process for preparing an amino acid in accordance with a series of coupled reactions wherein the substrate molecule is deaminated, deacetylated and reductively aminated to produce the amino acid. The substrate can be selected, or synthesised, so as to yield the corresponding amino acid, in this case specifically L-phenylalanine, but not limited to the latter.

This present invention relates to a novel process of converting acetamidocinnamic acid (ACA) to L-phenylalanine in high yields by maximising flux of carbon and nitrogen moieties, from said acetamidocinnamic acid substrate, through the coupled enzymatic reactions to L-phenylalanine product. Microorganisms which are capable of carrying out the latter transformation are described within. In particular, the present invention describes the complete metabolic pathway of acetamidocinnamic acid transformation in one particular strain of

*Corynebacterium equi* EVA-5.

The present invention essentially describes the use of a specific, inducible, cellular membrane permease (or translocase) which facilitates rapid uptake of acetamidocinnamic acid molecules from any surrounding medium and constitutes the first enzyme of the series of coupled enzymatic reactions (see Scheme 1 in Figure 1). The selective permease is required for whole cell biotransformations only and not essential for use of this invention as permeabilised cells, cell extracts, cell-free systems or a partially-purified enzyme preparation. Uptake of the acetamidocinnamic acid molecules, by whole cell systems of the aforementioned microorganisms, promotes synthesis (induction) of a deaminase enzyme which catalyses the conversion of acetamidocinnamate to alpha-aminocinnamic acid and acetic acid. The former intermediate is naturally unstable and converts to alpha-iminocinnamate which converts to the more stable keto form of phenylpyruvic acid and releases ammonium ions (see Scheme 1).

The present invention further relates to a novel process of L-phenylalanine synthesis by a coupling of the deaminase reaction to a dehydrogenase system and, to a lesser extent, to a transaminase enzyme. The phenylpyruvate generated is reaminated by a constitutive transaminase, which utilises endogenous amino acid pools as amino donors to produce L-phenylalanine. This activity

is, however, not the dominant or key enzyme in this present invention. Rather, a single induced dehydrogenase system is synthesised to carry out the reductive amination of phenylpyruvic acid to L-phenylalanine. The latter enzyme, hereby referred to as phenylalanine dehydrogenase can be induced by D-, L- or D,L-phenylalanine and to a lesser extent by L-tyrosine, L-histidine and other amino acids. The phenylalanine dehydrogenase described within requires a reduced cofactor for prolonged activity and continual L-phenylalanine synthesis (see Scheme 1).

It is always part of this invention to describe the coupling of a further dehydrogenase enzyme system to the key phenylalanine dehydrogenase so that reduced cofactors are always supplied as necessary for maintenance of phenylalanine production. Such coupled dehydrogenases can include lactate, pyruvate, alpha-keto-glutarate and formate dehydrogenases. All are relatively cheap sources of generating reducing equivalents and are described in detail within the concept of this present invention. The invention further relates to the use of these dehydrogenases in whole cells, cell-permeabilised extracts, cell-free extracts, pure enzyme mixtures etc., where addition of reducing power can be made directly in the form of nicotinamide adenine dinucleotides (NADH, NADPH) as the reduced compound or indirectly as lactate, pyruvate, formate, oxaloacetate, citrate or glucose or other energy sources common to those briefed in

state-of-the-art. It is also included in this present invention to provide reducing power to drive the active transport mechanism of the acetamidocinnamic acid permease, i.e. the first enzyme of the coupled sequence of enzyme-catalysed reactions described within (see Scheme 1).

It is also the intention of this present invention to describe a supplementary pathway of acetamidocinnamic acid metabolism via a double bond reduction by an acetamidocinnamate reductase enzyme to yield N-acetyl-L-phenylalanine. This stable intermediate is rapidly converted to L-phenylalanine by an efficient and constitutive deacetylase enzyme. It is also the intention of this present invention to describe the latter coupled enzyme pathway as a minor route to L-phenylalanine (see Scheme 1).

The complete metabolic pathway cited above and described in detail within therefore constitutes a novel and improved process for converting acetamidocinnamic acid to L-phenylalanine.

It is also an object of this present invention to increase the overall rate of reaction and yield of L-phenylalanine from the enzyme systems described within. The latter is achieved by optimising addition of rate-limiting intermediates; such additions include $NH_4^+$ ions, $NH_4OH$, $(NH_4)_2SO_4$, ammonium formate, phenylpyruvic acid, pyruvate, lactate, formate, NADH, $NAD^+$, acetate, oxaloacetate, alanine and isoleucine.

## DETAILED DESCRIPTION OF THE PREFERRED EMBODIMENTS

In accordance with the process of the present invention, L-phenylalanine is produced in high yields and at a high rate of conversion by utilising a sequence of enzymatic reactions present in certain natural strains of microorganisms. Such natural strains include species from the genera *Corynebacterium*, *Brevibacterium*, *Pseudomonas*, *Arthrobacter*, *Bacillus*, *Alcaligenes* and *Micrococcus*.

Examples of the novel isolates useful in the present invention are those hereinafter designated *Pseudomonas* EVA-1, *Bacillus* EVA-3, *Alcaligenes* EVA-4, *Corynebacterium* EVA-5 and *Coccoid* EVA-14 (possibly *Micrococcus* EVA-14). These strains were initially isolated from samples of soil and sewage in the area of Orangeville, Ontario, Canada.

### (i) Description of the novel isolate Corynebacterium EVA-5

In its characteristics, it is a gram positive rod of variable length, occurring mostly as short clubbed rods (almost coccoid) in palisades and 'v' forms. On testing with carbohydrate substrates, it is found that it ferments glucose and does not oxidise it. No acid was produced from any tested carbohydrate. The isolate strain grows between $18^{o}C$ and $37^{o}C$, but grows very best at $25^{o}C$-$30^{o}C$. It grows well at any pH from 4-9.

Accordingly the isolate probably belongs to the genus Corynebacterium and resembles the species Corynebacterium equi. It differs from the equi species, however, in that it does not reduce nitrate, its colonies are not pink in colour, it does not grow well above 30$^{o}$C, it does not exist as long curved rods in culture, and it can utilize ACA as sole carbon and nitrogen source for growth. Accordingly, it shows many of the characteristics of a number of species belonging to the genus Brevibacterium, and may be classifiable as such.

Hence it is concluded that the novel isolate strain is probabaly but not certainly a new species of Corynebacterium or a variety of the species Corynebacterium equi. For convenience, it is herein designated Corynebacterium strain EVA-5.

A viable culture of the strain Corynebacterium EVA-5 as described herein has been deposited with ATCC for storage therein under conditions giving permanence to the culture in viable form, and has been allotted accession reference no. 53208.

As regards its growth characteristics, the strain herein designated Corynebacterium EVA-5 grows well on a range of carbon and nitrogen sources, salts and extracts. A typical medium contains glucose, sucrose, fructose or glycerol at 1 - 5% w/v; $(NH_4)_2SO_4$, glutamate or asparagine, 0.2 - 1%; $KH_2PO_4$, 0.2 - 1%; $K_2HPO_4$, 0.1 - 0.4%; $Na_2HPO_4$, 0.05 - 0.02%; $MgSO_4.7H_20$, 0.01 - 0.07%;

NaCl, 0.1 - 1%; $CaCl_2.6H_2O$, 0.0001 - 0.01%; $FeCl_3.7H_2O$, 0.0001 - 0.0008%; $ZnSO_4.7H_2O$, 0.0001%; $MnCl_2$, $CuSO_4$, $CoCl_2$, in trace amounts; yeast extract, peptone or meat extract, 0.2 - 2%. Growth is stimulated by addition of various vitamins, particulary thiamine, biotin, pyridoxal, nicotinic acid, pantothenate and p-aminobenzoic acid, 1-45 $ugl^{-1}$. The strain can therefore be cultivated on a range of conventional laboratory and commercial media. The characteristics of Strain EVA-5 may be summarized as follows:

<u>Morphology</u>: Rods, 0.5 - 1.0 by 1.0 - 1.5 um, gram positive. Almost coccoid, non motile, pallisade and "V" forms. Non spore forming colonies cream coloured, circular shiny and mucoid. Black colonies formed on potassium tellurite.

<u>Physiology:</u>

| | |
|---|---|
| Nitrate reduction | - |
| Catalase | + |
| Oxidase | - |
| Capsule | + |
| Spore | - |
| Metachromatic granules | + |
| Acid Fast | - |

| | |
|---|---|
| Hemolysin | - |
| Starch hydrolysis | - |
| Gelatin hydrolysis | - |
| Urease | - |
| Casein hydrolysis | - |
| Lipolysis | + |

Acid from:

| | |
|---|---|
| Maltose | - |
| Galactose | - |
| Lactose | - |
| Glycerol | - |
| Arabanose | - |
| Fructose | - |
| Rhamnose | - |
| Xylose | - |
| Glucose | - |
| Melibiose | - |
| Sucrose | - |
| Erythrose | - |
| Sorbitol | - |

| | |
|---|---|
| Optimum temperature | 25$^{o}$C |
| Optimum pH | 5-8 |

The strains *Pseudomonas* EVA-1, *Bacillus* EVA-3,

Alcaligenes EVA-4 and Micrococcus EVA-14 were assigned to their respective genera by tentative identification using Bergey's Manual of Determinative Bacteriology, 8th Edition.

The active biochemical pathway which effects the conversion of ACA to L-phenylalanine in the process of the present invention is an enzyme or a sequence of enzymes produced by the novel microbial isolates exemplified by Corynebacterium EVA-5. These enzymes are maximally active in cells cultivated in any medium in the presence of ACA itself. Additions of ACA to growing cultures of the microorganisms, e.g. in amounts of from about 0.001% to about 10% w/v, induces the synthesis of the enzyme products.

For optimum conversion of ACA to L-phenylalanine in the biotransformation process of the present invention, it is preferred to maintain a cell concentration of the chosen strain of from about 5 to about 150 g/l cell dry weight. The pH range of the medium is preferably from about 4.5 to about 11 and optimally from 5 to 8. The process is preferably conducted in aqueous liquid growth medium containing appropriate nutrients, salts, buffers, etc., at temperatures in the approximate range 18°C to 50°C, and optimally from 25°C to 37°C. Aqueous phosphate buffers are suitably used in the biotransformation medium.

When conducted as described herein, using the preferred condition and procedures, conversion yields from

55 to 100% on a molar basis, and from 30 to 85% on a substrate weight basis, can be achieved.

The biotransformation process of the present invention is suitably preceded by a fermentation stage in which the cells of the chosen strain are cultivated, in a liquid growth medium containing an energy source e.g. sugar, a nitrogen source and salts as previously described. A small amount of ACA may be added to the cultivation medium, to stimulate synthesis of the appropriate enzyme or enzymes. The cells are preferably harvested at their maximum activity for enzyme production, and substrate ACA applied thereto for biotransformation to L-phe, under appropriately buffered conditions. The biotransformation process can also be carried out using stationary phase cells at the end of fermentation, by addition of concentrated ACA to the fermenter. Phenylalanine can then be recovered from the fermentation broth. Whole cells, free or immobilized, may be used in the biotransformation process. Alternatively, cell extracts containing the appropriate enzymes, or the enzymes themselves may be used. The cell, extracts or enzymes may be free or immobilized, e.g. by entrapment in solid beads, foams, attached to substrates and packed in flow columns, etc., all according to standard, known techniques of biotransformation processes.

In accordance with a preferred embodiment of the present invention a microorganism capable of synthesising

the appropriate system of enzymes is grown in a suitable medium. The media required are well-known by those of ordinary skill in the art and include, for example, media consisting of glucose, yeast extract, peptone, $(NH_4)_2SO_4$ and mineral salts. Other components can include glycerol, sucrose, glutamate, lactate, phenylalanine, citrate and sorbitol. Details of a typical medium example are mentioned above for novel strain Corynebacterium equi EVA 5.

The cells are cultivated within a temperature range of 25°C to 40°C, preferably 30°C, and between pH 4 and 9, preferably pH 7, and for 10 hours to 72 hours, preferably 32 hours, and when the cell concentration is between 5 $gl^{-1}$ and 40 $gl^{-1}$, preferably 25 $gl^{-1}$. The cells may be utilised for phenylalanine production in the presence of the growth medium, during or at the end of growth, or harvested by centrifugation or filtration to be used in a separate reactor and medium. The cells may be utilised intact or may be permeabilised or treated to release the enzymes required for the transformation. Such techniques include use of surfactants e.g. triton, cetyltrimethylammonium bromide, cetylpyridinium chloride, toluene, ethanol, xylene, sonication, freeze-thawing etc., and are all well-known to those familiar in the art. It has been found that if whole cells are used in accordance with procedures of this present invention, the cells are freely permeable to acetamidocinnamate, phenylpyruvic

acid, phenylalanine, pyruvate, lactate and other parts of the preferred embodiments of this present invention. In accordance with the procedure of this present invention it is preferred not to permeabilise cells prior to transformation reactions.

In order to maximise the yield of phenylalanine from the present invention, it is advisable to fully induce synthesis of all parts of the transformation system described within this invention. The short metabolic pathway from acetamidocinnamic acid uptake to L-phenylalanine excretion can be fully expressed if the cells are grown in an appropriate medium capable of sustaining growth and induction of synthesis of the individual enzyme components in the transformation sequence. Typically effective medium inducers include phenylalanine, lactate, pyruvate, citrate, sorbitol, formate, histidine, and acetamidocinnamic acid. Combinations of these components in standard carbohydrate and nitrogen salt-based fermentation media are also effective in maximising synthesis of the enzymes described in this invention.

The sequence of enzymatic reactions catalysing the transformation of acetamidocinnamate to L-phenylalanine in accordance with this present invention includes a specific (inducible) permease or membrane translocase system for transport of substrates into intact cells (e.g. acetamidocinnamate can utilise this protein

for more efficient uptake). The molecules of acetamidocinnamic acid are effectively deaminated to release ammonium ions, acetic acid and phenylpyruvic acid, via a series of naturally unstable intermediates such as alpha-aminocinnamate and alpha-iminocinnamate. The deaminase system described in this present invention is composed of two sub-units, each of molecular weight 37,000, and is induced when acetamidocinnamic acid is included in the growth medium. The products of this reaction are all utilised to effectively synthesise L-phenylalanine. The latter reaction is catalysed by a phenylalanine dehydrogenase enzyme which reaminates the phenylpyruvic acid with $NH_4^+$ and requires a reducing equivalent as cofactor. The acetate generated by the deaminase can supply this reduced cofactor upon primary metabolic events within the cell. All parts of the metabolic transformation pathway described in this present invention are synthesised naturally by the genes contained within the microorganisms described within.

In accordance with a preferred embodiment of this invention intact, fully-induced cells are removed from the growth medium and resuspended in a suitable buffer within a pH range of 5 to 8, preferably 6 to 7. The cells are held in suspension at a low temperature, preferably between $0^{o}C$ and $4^{o}C$ to preserve activity of the enzymes. Aliquots of suspension are added to reaction mixtures normally contained in the same buffer and pH and

containing various concentrations of the substrate, acetamidocinnamic acid. The reaction mixture containing cells is preferably reacted for 10 hours to 24 hours, at $34^{o}$ to $37^{0}C$ and pH 6 to 7. The acetamidocinnamate concentration is preferably in the range of 5 $gl^{-1}$ to 40 $gl^{-1}$ and cell concentrations between 2 $gl^{-1}$ and 100 $gl^{-1}$ dry weight, preferably 25 $gl^{-1}$.

In order to increase the yield of phenylalanine by overcoming product inhibition effects, permeabilising agents of the sort already described, and familiar to those in the art, can be added during the transformation. It is also in accordance with this invention but not the preferred methodology, to add substrates directly to intact cells suspended in the same growth medium with pH and temperature adjustments.

In accordance with the process of the present invention, whole cells can be disrupted using a variety of mechanical and enzymatic means familiar to those skilled in the art. The cell slurries can be clarified by centrifugation and filtration to produce clear cell-free extracts. The extracts can be purified through any number of steps, such as ammonium sulphate fractionation, gel filtration and ion exchange chromatography. The enzymes which form part of the transformation sequence in accordance with this invention can then be separated or pooled in purified mixtures according to the process of the invention to make L-phenylalanine. All of the

aforementioned extracts and enzymes can be added directly to reaction mixtures in buffers containing stabilising agents such as sorbitol, glycerol, dithiothreitol, polyethylene glycol, glutaraldehyde, phenylmethylsulphonyl fluoride, PMSF, gelatin, starch, sucrose and alginate. The latter can be run as non-crosslinked slurries to facilitate rapid transport of substrates to enzymes or as cross-linked matrices, e.g. entrapment as alginate beads. Extracts or enzymes can also be adsorbed, cross-linked or covalently bound to other materials to promote immobilisation of the parts of the preferred embodiments of this invention. The whole cells, extracts or enzymes can therefore be used in fluidised reactors or packed as beads, cubes, foams etc., into bioreactor columns of optimal design and are in accordance with the process of the present invention.

Another embodiment of the invention relates to the growth of cells on a medium containing a precursor or inducer to parts of the transformation system already described. Growth in the presence of acetamidocinnamic acid itself will induce synthesis of a permease protein to increase uptake of substrates and synthesis of a specific deaminase (dehydratase) enzyme to cleave the substrate to yield phenylpyruvic acid and synthesis of a phenylalanine dehydrogenase enzyme to reaminate the intermediate to yield phenylalanine. The latter enzyme can be further induced by including various forms and analogues of

phenylalanine in the growth medium; other amino acids also increase synthesis of this enzyme but to a lesser extent e.g. histidine. Another novel embodiment of this present invention relates to growth of the microorganism on a carbon source capable of producing excess reducing equivalents. Typically used substrates include lactate, pyruvate, citrate and formate. Cells grown in the presence of these carbon sources contain increased levels of the enzymes required to catabolise the substrate. Lactate, pyruvate and formate dehydrogenases are typically induced in this manner.

In accordance with the process of the present invention, whole or permeabilised cells, extracts or purified preparations are resuspended in a suitably buffered reaction mixture containing acetamidocinnamic acid and a number of effectors. A preferred embodiment of this invention is to add pyruvic acid or lactic acid to suitably induced cells in a reaction mixture. In this way the rate and overall yield of phenylalanine is markedly increased by replenishing the pool of reduced cofactors (NADH) used to convert the phenylpyruvic acid to phenylalanine. A further preferred embodiment of this invention is the addition of ammonium ions to reaction mixtures to maintain a constant pool of intracellular $NH_4^+$ ions, the cosubstrate for the phenylalanine dehydrogenase reaction. Ammonium ions can be added in the form of a salt, $NH_4Cl$ or $(NH_4)_2SO_4$ or as $NH_4OH$ or ammonium formate.

The addition of ammonium formate is preferred as both free $NH_4^+$ ions and reduced cofactor (NADH) can be generated with no accumulation of inhibitory coproduct: $CO_2$ is the only coproduct evolved.

The following worked Examples are intended to further illustrate the present invention and are not intended to limit the scope of this present invention.

Example 1:

Corynebacterium EVA-5 was grown in a medium containing ($gl^{-1}$); $KH_2PO_4$, 7; $Na_2HPO_4$, 2; $MgSO_4$, 0.4; NaCl, 2; $CaCl_2$, $6H_2O$, 0.01; $FeCl_3$, $7H_2O$, 0.08; $ZnSO_4$, $7H_2O$, 0.0001; $(NH_4)_2SO_4$,2; yeast extract 1; glucose 10. The medium was adjusted to pH 7.0 with 5M. NaOH, and sterilized by autoclaving.

Acetamidocinnamic acid (ACA) at pH7 was filter sterilized and added to the cooled medium to give 5 $gl^{-1}$. The medium was distributed in 1 litre volumes into 5 litre shake flasks and as 100 ml volumes in 500 ml flasks. A loopful from a slant of EVA-5 was inoculated into 100ml of the above medium and grown at 30°C, with shaking at 200 rpm, for 24h. A 5 litre shake flask was then inoculated with 50 ml of the seed culture and incubated under the same conditions. The whole culture was harvested, after 38h growth, using centrifugation.

The cell paste was washed in 50 mM phosphate

buffer pH 7.0 and resuspended in 80 ml of reaction mixture containing 1.5% ACA in 100 mM Tris buffer at pH 7.5 (adjusted with NaOH). This mixture was incubated at 30°C for 30h with shaking at 180 rpm. The cells were then separated from the reaction mixture and the L-phenylalanine content in the supernatant determined by paper chromatography. Under these conditions a final concentration of 4.8 $gl^{-1}$ of L-phenylalanine was produced.

Example 2:

The medium and cultivation procedure of Example 1 was repeated using *Corynebacterium* EVA-5. The cell paste, however, was resuspended in a 1.5% ACA reaction mixture adjusted to pH 6.2 (using 5M HCl). The reaction was carried out for 30h at 30°C and the final L-phenylalanine concentration obtained was 6.8 $gl^{-1}$.

Example 3:

The growth medium, cultivation and reaction conditions, using *Corynebacterium* EVA-5, were described in Example 2, except that the pH of the reaction was adjusted to 6.0 and the incubation carried out at 37°C. Under these conditions a final L-phenylalanine concentration of 9.8 $gl^{-1}$ was obtained.

Example 4:

All the conditions of Example 3 were repeated using strain EVA-5, except that the reaction was carried out at 34°C. A final concentration of 10.1 $gl^{-1}$ of L-phenylalanine was obtained.

Example 5:

*Corynebacterium* EVA-5 was grown in a medium containing, ($gl^{-1}$): yeast extract, 10; peptone, 10: meat extract, 5: NaCl 5; glucose 10. The medium was adjusted to pH 7.0 (using NaOH) and autoclaved for 15 mins.

ACA was added separately to a final concentration of 2 $gl^{-1}$. A litre volume of this medium was inoculated with 50 ml of 24h seed culture containing the same medium. The flask was grown at 30°C for 35h and harvested as described in Example 1. A thick cell suspension was prepared containing 6.2 g cell dry weight in 25 ml of 100 mM phosphate buffer pH 6.0. This suspension was added to 25 ml of a 2% ACA solution in 100 mM phosphate buffer. This incubation, containing 124 mg $ml^{-1}$ of cells, was shaken at 34°C for 16h at 190 rpm. The cells were then separated by centrifugation and the L-phenylalanine concentration in the supernatant determined to be 8.3 $gl^{-1}$. This calculates to a molar yield of 100% from the ACA in the incubation. No ACA was

found to be remaining in the supernatant.

Example 6:

The growth medium was prepared as in Example 5 and distributed as 100 ml aliquots in 500 ml shake flasks. After sterilization, and addition of ACA to 2 $gl^{-1}$, the flasks were inoculated with loopfuls from slants of *Pseudomonas* EVA-1, *Bacillus* EVA-3, *Alcaligenes* EVA-4 and *Micrococcus* EVA-14.

The cells were harvested and incubation conditions prepared as described in Example 5, except that reactions were carried out at pH 7.0 and 30°C. The reactions were terminated by centrifugation after 72h. The amounts of L-phenylalanine produced were as shown in Table 1.

TABLE 1

| Isolate | L-Phenylalanine Produced ($gl^{-1}$) after 72h |
|---|---|
| EVA-1 (*Pseudomonas*) | 2.2 |
| EVA-3 (*Bacillus*) | 3.7 |
| EVA-4 (*Alcaligenes*) | 5.5 |
| EVA-14 (*Micrococcus*) | 1.9 |

Example 7:

Slants of isolates *Pseudomonas* EVA-1, *Bacillus* EVA-3, *Alcaligenes* EVA-4, *Corynebacterium* EVA-5 and *Micrococcus* EVA-14 were used to inoculate 100 ml of medium containing ($gl^{-1}$): glucose, 10; peptone, 10; yeast extract, 10; meat extract 5; NaCl 5 and ACA 2 $gl^{-1}$. After 24h growth at 30°C and 200 rpm, these cultures were used to inoculate 1 litre of medium described in Example 5. A 10% inoculum was used. These cells were harvested after 40h growth at 30°C and washed in 0.9% NaCl. The cell pastes were resuspended in reaction medium containing: ACA 10 $gl^{-1}$ and glutamate 20 $gl^{-1}$, as amino donor, in 100mM phosphate buffer pH7.5. These flasks were shaken at 30°C, 180 rpm, for 72h. The amounts of L-phenylalanine produced are shown in Table 2.

TABLE 2

| ACA Isolate | L-Phenylalanine Produced ($gl^{-1}$) |
|---|---|
| EVA-1 (*Pseudomonas*) | 2.2 |
| EVA-3 (*Bacillus*) | 5.7 |
| EVA-4 (*Alcaligenes*) | 6.4 |

| | | |
|---|---|---|
| EVA-5 | (Corynebacterium) | 1.7 |
| EVA-14 | (Micrococcus) | 1.4 |

Example 8:

Corynebacterium EVA-5 was grown in 1 litre of the medium described in Example 1. The cells were harvested after 32h growth and washed in 0.9% NaCl. An incubation was prepared, according to the procedures already outlined in Example 1 to 7, containing 3.8 gm of cells (dry weight) in a final volume of 20 ml of tap water at pH 6.0 containing 140mg of acetamidocinnamate. The reaction was carried out in a 250 ml conical flask at 200 rpm shaking for 5h at 37°C. The total amount of L-phenylalanine produced was 96 mg, which calculates to a molar conversion yield of 84% in 5h.

Example 9:

Corynebacterium EVA-5 was grown in the medium described in Example 1, except that a variety of carbon and nitrogen sources were substituted for glucose and $(NH_4)_2SO_4$ in the medium.

Each medium was distributed in 100 ml aliquots in 500 ml flasks and sterilized. ACA was added separately to a final concentration of 2 $gl^{-1}$. Each flask was

inoculated with 10% inoculum from seed cultures as in Example 1. After 32h growth the cultures were harvested and washed in 0.9% saline before resuspending the cell paste in 10 ml of reaction medium containing: 1% ACA in 100 mM phosphate buffer pH 7.5 and incubated at 30°C for 30 h. The amounts of L-phenylalanine produced are shown in Table 3.

TABLE 3

| Growth Medium | | Biotransformation |
| --- | --- | --- |
| Carbon Source | Nitrogen Source | L-Phenylalanine Produced ($gl^{-1}$) |
| Glucose | $(NH_4)_2SO_4$ | 5.7 |
| Glucose | Urea | 3.8 |
| Glucose | Asparagine | 5.2 |
| Glucose | Apartate | 5.7 |
| Glucose | Glutamate | 4.4 |
| Sucrose | $(NH_4)_2SO_4$ | 4.9 |
| Glycerol | $(NH_4)_2SO_4$ | 6.1 |
| Glutamate | $(NH_4)_2SO_4$ | 4.4 |
| Fructose | $(NH_4)_2SO_4$ | 5.5 |
| Glucose | Acetamidocinnamate | 5.1 |
| Acetamidocinnamate | $(NH_4)_2SO_4$ | 5.5 |
| Acetamidocinnamate | - | 4.8 |

Example 10:

An Agar slope of natural isolate Corynebacterium equi EVA-5 was used to inoculate 50 ml of ACA induction medium I containing ($gl^{-1}$): glucose 10, meat extract 10, peptone 10, yeast extract 5, NaCl 2, $(NH_4)_2SO_4$ 2 and acetamidocinnamic acid 2 $gl^{-1}$. The medium was adjusted to pH 6.8 with concentrated HCl and sterilised by autoclaving at 121°C for 15 minutes. The glucose and acetamidocinnamate were added after autoclaving by sterile filtration. The culture was grown at 30°C, in a 250 ml conical flask,with shaking at 162 rpm for 24 hours. The whole contents of this flask were used to inoculate 500 ml of ACA induction medium I in a 3-litre shake flask. Cells were harvested after 30 hours growth and washed twice in 100 nM phosphate buffer pH 7.0 via centrifugation. The cells were resuspended in the phosphate buffer to a concentration of 200-300 mg wet cell weight per ml and stored at 4°C, on ice, until use.

An aliquot (10 ml) of cell suspension was mixed with 10 ml of a solution containing 20$gl^{-1}$ acetamidocinnamic acid in 100 mM phosphate buffer pH 6.8. The mixture was incubated at 34°C in a 100 ml conical flask and shaken at 140 rpm for 24 hours. The resulting mixture contained L-phenylalanine at a concentration of 5.4$gl^{-1}$ as determined by paper chromatography.

Example 11:

An aliquot (20 ml) of cell suspension, prepared as in Example 1, was mixed with glass beads (0.25 u) in a 1:1 ratio and subjected to a total of 5 minutes disruption in a Braun shaker (with chilling). The cells and debris were removed by centrifugation at 20.000 g (SS34 rotor) for 20 minutes and the extract filtered through tissue paper and stored on ice. An aliquot (4 ml) of cell-free extract was incubated with 4 ml of a 10 $gl^{-1}$ acetamidocinnamate solution at $34^{o}C$ for 24 hours. The resulting mixture contained 4.1 $gl^{-1}$ L-phenylalanine calculating to a molar conversion yield of 99%.

Example 12:

An aliquot (2 ml) of the cell-free extract, prepared as described in Example 11, was mixed with 2 ml of the following solutions: acetamidocinnamic acid (ACA) 20 mg $ml^{-1}$ only; ACA 20 mg $ml^{-1}$ + $(NH_4)_2SO_4$ 10 mg $ml^{-1}$; ACA 20 mg $ml^{-1}$ + glutamic acid 10 mg $ml^{-1}$; ACA 20 mg $ml^{-1}$ + L-alanine 15 mg $ml^{-1}$; ACA 20 mg $ml^{-1}$ + pyruvate 15 mg $ml^{-1}$. All solutions were in 100 mM phosphate buffer at pH 6.8 and incubated at $37^{o}C$ for 24 hours. The resulting mixtures respectively yielded 3.0, 5.8, 6.1, 6.5 and 6.8 $gl^{-1}$ L-phenylalanine.

Example 13:

An agar slope of Corynebacterium equi EVA-5 was used to inoculate 50 ml of ACA Induction Medium I, as described in Example 10. This flask was incubated at 30°C for 24 hours and used to inoculate 500 ml of a medium containing ($gl^{-1}$): $KH_2PO_4$ 7, $Na_2HPO_4$ 2, $MgSO_4.7H_2O$ 0.4, $CaCl_2.6H_2O$ 0.01, $FeCl_3.6H_2O$ 0.008, $ZnSO_4.7H_2O$ 0.0001, NaCl 2, $(NH_4)_2SO_4$ 2, yeast extract 5, acetamidocinnamic acid 2 and glucose $10gl^{-1}$. The pH of the medium was adjusted to 7.0 with 10 M HCl and sterilised by autoclaving. The trace salts, ACA and glucose were autoclaved separately and added later. The culture was grown for 30 hours at 30°C, harvested by centrifugation, washed twice in phosphate buffer and resuspended in the same buffer and stored on ice.

Example 14:

Media were prepared as described in Example 13 but contained different carbon sources with and without acetamidocinnamic acid present. Cultures were grown and cell suspensions prepared according to Example 13. An aliquot (10 ml) of suspension, from each medium, was mixed with 10 ml of a 40 $gl^{-1}$ solution of acetamidocinnamate in 0.1M phosphate buffer pH 6.0. The mixtures were incubated at 37°C for 24 hours with shaking at 142 rpm in 100 ml

conical flasks. The following yields of L-phenylalanine were obtained (Table 4).

TABLE 4

| Medium | L-Phenylalanine [$gl^{-1}$] |
|---|---|
| Glucose/NO ACA | 1.2 |
| Glucose + ACA | 5.9 |
| L-Phenylalanine/NO ACA | 3.0 |
| L-Phenylalanine + ACA | 9.6 |
| Acetamidocinnamic acid/no glucose | 7.2 |
| Acetamidocinnamic acid/ L-Phe.(N-source) | 9.2 |

Example 15:

Cultures were set up and cells harvested as is described in Example 13 except medium contained no acetamidocinnamic acid inducer. The inducer was added, via sterile filtration, to a final concentration of $2gl^{-1}$, at times 0 hour, 16 hours and 24 hours. Cells were harvested before and after addition and these were reacted with 2% acetamidocinnamate in 100 mM phosphate buffer pH 6.0 as described in Example 10. The production of L-phenylalanine

to $6gl^{-1}$ was taken as 100% in the Table below (Table 5).

TABLE 5

| Addition of ACA | Cell Harvest Time | L-Phenylalanine yield (%) Culture 1 | Culture 2 | Culture 3 |
|---|---|---|---|---|
| 0 hour | 16 hours | 28 | - | - |
| | 24 hours | 60 | - | - |
| | 40 hours | 100 | - | - |
| 16 hours | 16 hours | - | 6 | - |
| | 24 hours | - | 70 | - |
| | 40 hours | - | 120 | - |
| 24 hours | 24 hours | - | - | 8 |
| | 32 hours | - | - | 60 |
| | 48 hours | - | - | 105 |

Example 16:

Cells of Corynebacterium equi EVA-5 were grown in ACA induction medium I (8 litres) at 30°C for 32 hours. The cells were harvested and washed in 100 mM Tris-HCl pH 7.0 and resuspended in the same buffer to form a thick cell paste. The cells were disrupted by a 2 x 15 minute run in a Dyno Mill and cells and debris removed by

centrifugation at 19,000 rpm (SS34 rotor). The clear cell-free extract was filtered through tissue and loaded onto a column of Sephadex G50 and eluted using the same buffer. The fractions with highest protein content were pooled and used for subsequent studies to test the effect of various intermediates and cofactors on ACA transformation. From a range of metabolites tested, the following compounds were found to affect activity of the ACA transforming system. Activities were determined as mg of L-phenylalanine produced per ml per mg protein per hour from reactions containing 10 mg $ml^{-1}$ ACA and incubated at 37°C for 15 hours (see Table 6).

TABLE 6

| Metabolite | ACA ---▶ L-Phe Activity [%] |
|---|---|
| Control | 100 |
| ATP | 100 |
| NADH | 809 |
| NADPH | 255 |
| Cyclic AMP | 209 |
| $CaCl_2$ | 302 |
| $NH_4^+$ | 285 |
| Pyruvate | 423 |
| Oxaloacetate | 398 |

Example 17:

Cell-free extract of *Corynebacterium equi* EVA-5 was prepared according to methods described in Example 16. Aliquots (1 ml) were mixed with 1 ml reaction mixture containing 1% acetamidocinnamate in 100 mM Tris HCl pH 6.2 and varying concentrations of nicotinamide adenine dinucleotide reduced (NADH) form. The effect on L-phenylalanine production from ACA is shown below (Table 7).

TABLE 7

| | | | | | | |
|---|---|---|---|---|---|---|
| NADH (mM) | | 0 | 0.2 | 0.5 | 1.0 | 7.5 |
| ACA | L-Phe. Activity (%) | 100 | 118 | 198 | 305 | 320 |

Example 18:

Cell-free extract of *Corynebacterium equi* EVA-5 was prepared according to Example 16. Aliquots were mixed with a range of substrate solutions in combination with various effectors of the ACA transformations. Reactions were carried out at 37°C for 16 hours and L-phenylalanine produced estimated chromatographically. Well-known inhibitors, such as semicarbazide (SCZ)and aminooxyacetic acid (AOA), were included in these experiments. The results are shown in Table 8.

TABLE 8

| Reaction Mixture | L-Phenylalanine [mg $ml^{-1}$] |
|---|---|
| 1% Acetamidocinnamate (ACA) | 3.9 |
| 1% Phenylpyruvic acid (PPY) | 3.6 |
| 1% N-Acetyl-L-Phenylalanine (NACE) | 4.7 |
| 1% ACA + 1% $(NH_4)_2SO_4$ | 4.4 |
| 1% ACA + 1% Pyruvate | 6.1 |
| 1% ACA + 1% $(NH_4)_2SO_4$ + 1% Pyruvate | 5.8 |
| 1% ACA + 10 mM Aminooxyacetate (AOA) | 2.7 |
| 1% ACA + 10 mM Semicarbazide (SCZ) | 3.0 |
| 1% ACA + $(NH_4)_2SO_4$ + Pyruvate + AOA | 4.9 |
| 1% ACA + $(NH_4)_2SO_4$ + 1% L-Alanine + AOA | 6.3 |
| 1% PPY + 1% $(NH_4)_2SO_4$ | 4.1 |
| 1% PPY + 1% Pyruvate | 5.2 |
| 1% PPY + 1% $(NH_4)_2SO_4$ + Pyruvate | 4.9 |
| 1% PPY + AOA | 0.9 |
| 1% PPY + Semicarbazide | 1.1 |
| 1% PPY + $(NH_4)_2SO_4$ + Pyruvate + AOA | 3.9 |
| 1% N-Ace-Phe. + $(NH_4)_2SO_4$ | 5.4 |
| 1% N-Ace-Phe. + Pyruvate | 5.6 |
| 1% N-Ace-Phe. + AOA + SCZ | 6.4 |

Example 19:

Freshly induced cells of Corynebacterium equi EVA-5 were prepared according to methods described in Example 10. Cells were resuspended in 0.1M phosphate buffer and mixed with reaction solution to give a final concentration of 20 mg $ml^{-1}$ cell dry weight, 10 mg $ml^{-1}$ acetamidocinnamate and 12 mg $ml^{-1}$ of the permeabilising agents shown. The suspensions were incubated at 37°C for 16 hours and L-phenylalanine production monitored using chromatographic methods. Similar reaction mixtures were set up except ACA was omitted and cells were preincubated with permeabilising agents for 2 hours at 30°C. Suspensions were then disrupted using standard sonication techniques (3 x 30 sec. bursts) and cells and debris removed by high speed centrifugation. The cell free extracts (still containing permeabilising agents) were reacted with ACA as with whole-permeabilised cells. The results are shown in Table 9.

TABLE 9

| | L-Phenylalanine (yield %) | |
|---|---|---|
| Permeabilising Agent | Whole Cells | Cell-free Extract |
| Control | 100 | 100 |
| Triton-X | 37 | 128 |
| Cetylpyridinium chloride | 46 | 98 |
| Toluene | 43 | 103 |
| Xylene | 34 | 91 |

Phenylalanine production is decreased as cell membranes become disrupted.

Example 20:

Freshly induced cells of Corynebacterium equi EVA-5 were prepared according to methods described in Example 10. The cells were mixed with various reaction solutions to give a final concentration of 38 mg $ml^{-1}$ cell dry weight in 100 mM phosphate buffer pH 7.0. The reactions (30 ml) were carried out in 100 ml conicals at 37°C with shaking at 140 rpm for 24 hours. The concentration of L-phenylalanine produced and conversion yields are shown in Table 10.

TABLE 10

| Reaction Mixture | L-Phenylalanine [$gl^{-1}$] | Yield [%conversion] |
|---|---|---|
| Phenylpyruvate 0.5% + glutamate 2% + *PLP 0.1 mM | 2.1 | 42 |
| Phenylpyruvate 0.5% + Aspartate 2% + PLP 0.1 mM | 2.4 | 48 |
| Phenylpyruvate 0.5% + L-Alanine 2% + PLP 0.1 mM | 2.9 | 58 |
| Phenylpyruvate 1.0% + glutamate 2% + PLP 0.1 mM | 3.2 | 32 |
| Phenylpyruvate 1.0% + | | |

| | | |
|---|---|---|
| alanine 2% + PLP 0.1 mM | 3.9 | 39 |
| Phenylpyruvate 1.5% + glutamate 3% + PLP 0.1 mM | 4.9 | 30 |
| Phenylpyruvate 2.0% + glutamate 3% + PLP 0.1mM | 6.0 | 30 |

*PLP: pyridoxal phosphate

Example 21:

Cells of Corynebacterium equi EVA-5 were prepared as in Example 10 and reactions set up according to Example 20. In this case reaction mixtures contained varying concentrations of acetamidocinnamic acid in 100 mM phosphate buffer pH 7.0. The results are shown below in Table 11.

TABLE 11

| Reaction Mixture | L-Phenylalanine $[gl^{-1}]$ | Molar [conversion %] |
|---|---|---|
| Acetamidocinnamate 0.5% | 3.9 | 91.8 |
| Acetamidocinnamate 1.0% | 7.9 | 96.3 |
| Acetamidocinnamate 1.5% | 10.2 | 82.9 |
| Acetamidocinnamate 2.0% | 11.3 | 68.9 |

Example 22:

Cells of *Corynebacterium equi* EVA-5 were prepared as in Example 10 and resuspended in 100 mM Tris buffer pH 6.8 to a concentration of 34.2 mg $ml^{-1}$ dry weight. An aliquot (15 ml) of this suspension was mixed with 15 ml of a solution containing 20 mg $ml^{-1}$ ACA in 100 mM Tris buffer pH 6.8. The reaction was incubated at $37^{o}C$ for 24 hours and 1 ml samples removed periodically. Cells were rapidly removed by filtration and lysed using 20% perchloric acid. Extracts were neutralised with 6N NaOH and cell debris removed by rapid centrifugation. Using this method the intracellular pools of L-phenylalanine were determined and found to increase from 19mM (0 hour) to 40 mM (4 hours) to 67 mM (24 hours). The pools of acetamidocinnamate decreased correspondingly 51 mM (0 hour), 12 mM (4 hours) and 12 mM (24 hours).

Example 23:

Cells of *Corynebacterium equi* EVA-5 were grown and prepared according to methods described in Example 13. The cells were resuspended to a concentration of 180 mg wet wt. per ml buffer and mixed with equal aliquots of reaction solutions. The reactions were incubated at $37^{o}C$ for 24 hours and L-phenylalanine production monitored.

After 24 hours, the cells were removed by centrifugation, resuspended in a large volume of buffer and leached of endogenous L-phenylalanine for 6 hours at 30°C. Cells were then removed and resuspended in the same volume of reaction as in the first incubation. This procedure was carried out for several consecutive runs and the results shown below (Table 12).

TABLE 12

| Reaction Mixture | L-Phenylalanine ($mg\ ml^{-1}$) 4th Run (total = 96 hours) |
|---|---|
| Phenylpyruvate 1% (PPY) | 1.2 |
| PPY 1% + $NH_4Cl$ 1% + NADH 10 mM | 2.7 |
| PPY + $NH_4Cl$ + NADH + Cetylpyridinium chloride 1% | 4.9 |

Example 24:

Cells of *Corynebacterium equi* EVA-5 were prepared by methods described in Example 13. An aliquot (20 ml) of freshly induced cells was mixed with 20 ml buffered solution of ACA, 20 $gl^{-1}$, pH 6.2 and a similar aliquot with 20 ml of solution containing 2% ACA, 2% pyruvic acid and 0.5% $(NH_4)_2SO_4$ pH 6.2 in 100 mM phosphate buffer. Both reactions were incubated at 37°C for 24 hours and L-phenylalanine produced monitored chromatographically. After every 24 hour period the cells

were removed from used reaction mixtures, washed and leached for 5 hours in phosphate buffer and resuspended in the same volumes of the respective substrate mixtures. The continuous production of L-phenylalanine by these cells is shown below in Table 13.

TABLE 13

| | L-Phenylalanine [$gl^{-1}$] | | | | |
|---|---|---|---|---|---|
| Reaction Mixture | Run 1 | Run 2 | Run 3 | Run 4 | Run 5 |
| Acetamidocinnamate (ACA) 1% + 32 $gl^{-1}$ cell dry weight | 8.05 | 3.12 | 1.02 | 0.4 | 0 |
| ACA 1% + Pyruvate 1% + $(NH_4)_2(SO_4$ 0.5% + 32 $gl^{-1}$ cell dry weight | 8.91 | 8.03 | 5.22 | 6.57 | 4.93 |

Example 25:

A cell-free extract of *Corynebacterium equi* EVA-5 was prepared according to methods described in Example 16. Freshly induced cells of EVA-5 were prepared according to methods in Example 10. Aliquots (5 ml) of the extract and cell suspension were reacted separately with solutions containing ACA 1% and phenylpyruvate 1% as substrates with and without the pyridoxal phosphate-requiring enzyme inhibitor, aminooxyacetic acid (AOA) see Table 14.

TABLE 14

| Reaction Mixture | L-Phenylalanine [$gl^{-1}$/12 hrs.] |
|---|---|
| ACA 1% + AOA 0.1 mM + cell extract | 0.6 |
| ACA 1% + pyruvate 1% + cell extract | 5.4 |
| ACA 1% + pyruvate 1% + AOA 100 mM + cell extract | 3.9 |
| Phenylpyruvate 1% + AOA 100 mM + cell extract | 0.2 |
| Phenylpyruvate 1% + glutamate 3% + AOA 100 mM+ cell extract | 0.5 |
| PPY 1% + $(NH_4)_2SO_4$ 1% + pyruvate 2% + AOA 100 mM + cell extract | 2.7 |
| PPY 1% + $(NH_4)_2SO_4$ 0.5% + pyruvate 2% + AOA 100 mM + whole cell suspension | 4.4 |
| PPY 1% + $(NH_4)_2SO_4$ 0.5% + AOA 100 mM + whole cell suspension | 0.5 |

Example 26:

Cells of Corynebacterium equi EVA-5 were prepared by methods according to Example 13. Reaction mixtures contained a final cell concentration of 34 mg $ml^{-1}$ cell dry weight, 10 mg $ml^{-1}$ acetamidocinnamate in 200 mM phosphate buffer pH 6.2 and, where appropriate, 20 mg

$ml^{-1}$ of oxaloacetic acid and acetic acid. Reactions (50 ml) were incubated at 34°C for 24 hour runs and L-phenylalanine formed was estimated chromatographically. After each 24 hour run, the cells were removed by centrifugation, washed twice and leached of endogenous amino acids in phosphate buffer. The cells were then resuspended in the same volume of the respective reaction mixtures and the producedure of incubation continued for several runs, see Table 15.

TABLE 15

| Reaction Mixture | L-Phenylalanine [$gl^{-1}$] Run 1 | Run 2 | Run 3 | Run 4 |
|---|---|---|---|---|
| ACA 1% | 6.2 | 1.9 | 0.9 | 0.1 |
| ACA 1% + Oxaloacetate 2% | 7.9 | 6.9 | 4.2 | 5.4 |
| ACA 1% + Acetate 2% | 7.3 | 5.3 | 3.7 | 4.0 |

Example 27:

Cells of *Corynebacterium equi* EVA-5 were prepared according to Example 13. Reaction mixtures were prepared containing 25 mg $ml^{-1}$ cell dry weight, 100 mM phosphate buffer pH 6.2 and 10 mg $ml^{-1}$ acetamidocinnamic acid in a final volume of 20 ml. The reaction was incubated at 34°C for 24 hours and samples of cell-free

reaction mixture removed periodically. The supernatants were used to determine the concentration of phenylalanine and other components produced during the transformation. Samples (500 ul) were mixed with 200 ul of 28% w/v ferric chloride solution prepared in 10% v/v $H_2SO_4$ and incubated at 30°C for 3 minutes. Controls of standard keto acid solutions (including pyruvate, phenylpyruvate, oxaloacetate, alpha-ketoglutarate, alpha-ketocaproate, alpha-ketoisovalerate) were also incubated. Only phenylpyruvate solutions produced a positive colour from pale yellow to deep green. Reaction samples produced the same green coloration indicating the presence of phenylpyruvic acid. The latter keto acid was assayed again using the well-known 2,4-dinitrophenol hydrazine technique at 416 nm for keto acids. Samples were also dissolved in 5% NaOH to measure the equilibration of the enol:keto forms at 320 nm. The samples (0.5 ml) were dissolved in a solution (3 ml) of sodium arsenate (2M pH 6.2) and an identical sample mixed with 3 ml of a solution containing 1M boric acid in 2M sodium arsenate pH 6.2. The solutions were incubated at 25°C for 15 minutes and then the latter sample (formation of enol-borate complex) read against the free keto sample at 300 nm. All four assay techniques detected the presence of phenylpyruvic acid in increasing quantities throughout the ACA transformation.

Example 28:

Reactions, using cells of Corynebacterium equi EVA-5, were set up according to Example 27 and monitored over 12 hours. Phenylpyruvate produced was quantitated using the methods described in Example 27 and excretion of this keto acid was found to increase as the reaction progressed : PPY (time) : 0 (0 hour), 1.8 mM (2 hours), 3.6 mM (4 hours(, 4.8 mM (6 hours), 6.1 mM (8 hours), 9.9 mM (12 hours).

Example 29:

Cells of Corynebacterium equi EVA-5, were prepared according to methods described in Example 13. The cells were resuspended and disrupted using a dyno mill as described in Example 16. A total of 35 ml of cell-free extract was prepared from 20 g cell dry weight. The extract was fractionated using ammonium sulphate and the 40-70% fraction recovered and dialysed against 4 litres of Tris-HCl pH 7.5. This fraction was loaded onto a Sephacryl S-300 column (2 x 60 cm) and eluted with 50 mM Tris HCl at a flow rate 4 ml $h^{-1}$. The active fractions (6-11), assayed with ACA and estimating phenylpyruvate using 2,4-DNP methodology, were concentrated to 20 ml and loaded in 5 ml aliquots onto DEAE-Fractogel 650S and

equilibrated with Tris-HCl pH 7.5. The column was eluted with varying concentrations of NaCl in Tris buffer. The active fractions, 13-19, were concentrated and loaded onto a column of Sephacryl S300 (2.5 x 200 cm). The active fractions, 130-150, were loaded onto a DEAE-Fractogel 650S column and eluted with a 200 ml gradient of 0.2M NaCl at a flow rate of 50 ml per hour. Fractions 113-120 were concentrated and loaded onto a column of Sephacryl S200 and eluted with 50 mM Tris-HCl buffer, pH 7.5. A final gel filtration was carried out on Sephacryl S200 and the major protein/activity peaks between fractions 162-168 retained. The fractions were run on SDS slab electrophoresis and proteins stained with Coomassies Brilliant Blue R250. Only single protein bands were detected corresponding to a molecular size of 37,000. The protein was loaded onto Sephacryl S200, along with numerous protein standards, and eluted to give a molecular weight of 73,000. The enzyme (2 sub-units) was found to catalyse the removal of acetamidocinnamic acid from solution with the concomitant release of phenylpyruvic acid and ammonium ions. These products could be detected individually by specific reagents (2,4-DNP and phenol hypochlorite) and by HPLC and TLC scanner analysis. The enzyme was classified as ACA deaminase.

Example 30:

Cells of Corynebacterium equi EVA-5 were grown according to methods described in Example 13 but media contained glucose with and without 2 $gl^{-1}$ acetamidocinnamate as inducer. Two cell suspensions were therefore prepared, one constituting a fully-induced preparation the other non-induced cells with respect to ACA transformation. Disappearance of ACA from reaction mixture was measured by its specific absorbance at $A_{279}$ and confirmed using HPLC analysis. Phenylpyruvate was measured by methods described in Example 27 and L-phenylalanine by chromatographic technique. Results are shown in Table 16.

TABLE 16

| Product [$gl^{-1}$] | Non-Induced | | | | | | Induced | | | | | |
|---|---|---|---|---|---|---|---|---|---|---|---|---|
| | 0h | 2h | 4h | 6h | 8h | 24h | 0h | 2h | 4h | 6h | 8h | 24h |
| Acetamido-cinnamate | 0 | 0.2 | 0.1 | 0.3 | 0.7 | 2.4 | 0 | 1.2 | 2.5 | 4.1 | 5.5 | 9.4 |
| Phenyl-pyruvate | 0 | 0.1 | 0.1 | 0.05 | 0.05 | 0.3 | 0 | 0.34 | 0.68 | 0.9 | 1.16 | 0.1 |
| Phenyl-alanine | 0 | 0.2 | 0.1 | 0.4 | 1.0 | 2.0 | 0 | 0.65 | 1.4 | 1.8 | 3.1 | 6.5 |

Example 31:

Cells of Corynebacterium equi EVA-5 were grown according to Example 13 except glucose was omitted and lactate used as a carbon source for growth. ACA, when added, was at a concentration of $2gl^{-1}$. Reaction mixtures contained ACA 1% in 500 mM phosphate buffer pH 6.2. After 5 hours of reaction at 37°C, glucose-grown cells (+ACA inducer) produced 2.1 $gl^{-1}$ phenylalanine, lactate-grown cells (-ACA inducer) yielded 0.96 $gl^{-1}$ phenylalanine and lactate-grown cells (+ACA inducer) produced 7.4 $gl^{-1}$ phenylalanine.

Example 32:

Cells of Corynebacterium equi EVA-5 were grown as described in Example 13 except L-phenylalanine was used as sole carbon source for growth. Reactions were carried out for 5 hours at 34°C using 1% ACA in phosphate buffer at pH 6.2. Cells grown with glucose + ACA produced 1.9 $gl^{-1}$ L-phenylalanine whilst cells grown with L-phenylalanine + ACA yielded 7.9 $gl^{-1}$ L-phenylalanine. Cell-free extracts, of the cells prepared from L-phenylalanine growth media, were prepared according to Example 16. Addition of 5 mg $ml^{-1}$ ACA to extracts produced 2.1 $gl^{-1}$ L-phenylalanine after 8 hours at 37°C. Addition of 5 mg $ml^{-1}$ ACA plus

aminooxyacetic acid (200 mM) resulted in 0.13 mg $ml^{-1}$ L-phenylalanine. Addition of 5 mg $ml^{-1}$ ACA plus 200 mM aminooxyacetate plus 20 mM NADH resulted in 4.3 mg $ml^{-1}$ L-phenylalanine.

Example 33:

Cells of *Corynebacterium equi* EVA-5 were grown as described in Example 13. Non-induced cells were prepared by omitting ACA from the growth medium. Cell suspensions were washed three times in 200 mM phosphate buffer pH 6.2 and incubated at 30°C in buffer for 10 hours to leach out excess metabolites such as $NH_4^+$ ions. Cell-free extracts were also prepared according to methods described in Example 16. The extracts were dialysed against 4 litres of buffer for 10 hours prior to reaction. Reactions were set up containing 25 mg $ml^{-1}$ cell dry weight of induced and non-induced cells, 10 mg $ml^{-1}$ acetamidocinnamic acid in 200 mM phosphate buffer pH 6.2. Where cell-free extracts were used, the final concentration of protein was 8.5 mg $ml^{-1}$ (Bradford estimation). Reactions were carried out at 34°C for 24 hours and monitored at intervals. Ammonium ions were estimated using standard methods (Nesslers reagent, phenol-nitroprusside reagent, glutamate dehydrogenase reaction) and in reactions with non-induced cells and extracts, the concentrations were 0.002 mM (5 hours) and 0.05 mM (16 hours). In

reactions with induced cells and extracts, ammonium ion concentrations varying from 0.1 to 6.2 mM (20 hours) were detected. In the latter reactions there was also concomitant uptake of ACA and L-phenylalanine excreted as detected and described in previous Examples.

Example 34:

Cell-free extracts of Corynebacterium equi EVA-5 and a mutant strain, OAR1-16* of this parent, were prepared according to methods described in Example 16. The cells had been fully induced by growth of both EVA-5 and OAR1-16 on medium containing L-phenylalanine (1%) as carbon source and acetamidocinnamate (0.2%). Acetamidocinnamate deaminase was assayed by measuring the release of phenylpyruvate and phenylalanine dehydrogenase activity was measured by similarly monitoring release of phenylpyruvate ions. Results are shown in Table 17.

* C.equi mutant strain OAR1-16 has been deposited with ATCC under accession No. 53529. Details of its preparation and characterising data are given in the Appendix below.

TABLE 17

| Extract | Substrate/Enzyme | Activity [u moles $min^{-1}$ $ml^{-1}$] |
|---|---|---|
| EVA-5 | ACA / Deaminase | 18.7 |
| EVA-5 | L-phenylalanine / Dehydrogenase | 0.9 |
| OAR1-16 | ACA / Deaminase | 26.5 |
| OAR1-16 | L-Phenylalanine / Dehydrogenase | 23.0 |

A portion of the mutant, OAR1-16, extract was purified via ammonium sulphate fractionation, Sephacryl gel-filtration and DEAE cellulose affinity chromatography to obtain a preparation of this novel enzyme activity, phenylalanine dehydrogenase. Aliquots of the active fractions were incubated with L-phenylalanine and NAD+, as cofactor, at 34°C: phenylpyruvic acid was identified as the product accumulating in the reaction. Aliquots of the enzyme preparation were also incubated with phenylpyruvic acid, ammonium ions and NADH as cofactor: L-phenylalanine was identified as the product accumulating in the reaction mixture and phenylpyruvate concentration concomitantly decreased. The omission of either $NH_4^+$ or NADH resulted in no product formation. A mixture of these active fractions (phenylalanine dehydrogenase) and the pure, homogeneous preparation of deaminase described in Example 20, was able to catalyse the conversion of acetamidocinnamic acid, $NH_4^+$ ions and NADH to L-phenylalanine.

Example 35:

Cell-free reaction supernatants, from several of the types of reactions described in Examples 10 to 34, were used for derivatisation and total mass analysis using conventional gas chromatography - mass spectrometry methodology. A computer-based compound bank consisting of over 76,000 chemicals was used to screen and identify major components of reaction mixtures for intermediates and products resulting from acetamidocinnamic acid biotransformations of the sort previously described. Compounds were detected which precisely correlated with, or closely resembled, benzoic acid, phenylacetate, phenyllactate, cinnamic acid, hydroxystyrene, methyl indole acetate, aminocinnamic acid, phenacetaldehyde, phenylethylamine.

Example 36:

The product of the acetamidocinnamate transformations, via ACA deaminase and phenylalanine dehydrogenase, was identified using HPLC, paper and TLC chromatography, enzymatic assay as phenylalanine. The resolution of the amino acid product was carried out as follows: samples (10 ul) were injected onto a Chiralpak WH column (25 x 0.46 cm) in a Water HPLC assembly and compared to mixtures of pure, authentic standards of D-and L-isomers of phenylalanine. Using the equipment under conditions well-known to those familiar with the art of HPLC analysis, the D-isomer was separated first and clearly resolved within a retention time of 3.2 units from the L-isomer. Samples from all examples of ACA transformations described within were resolved in this way and found to contain 100% of the L-isomer of phenylalanine and no detectable D-phenylalanine. Only when N-acetyl-D-phenylalanine was used as substrate for deacetylase could D-phenylalanine be detected as product in the reaction.

# APPENDIX

## Production and Characteristics of C equi mutant strain OAR1-16

### Production

Cells of parent strain Corynebacterium equi EVA-5 (ATCC 53208) were used to derive the mutant OAR1-16 by the following procedure:

An LB agar slant of C. equi EVA-5 was used to inoculate 20 ml of LB broth in a 250 ml flask and grown for 24 hours at 30°C with shaking 160 rpm.

Cell suspensions of expontentially growing EVA-5 were mutagenised as follows: Ethyl methane sulphonate (EMS) was added to a final concentration of 1% (v/v) and shaken vigorously to disperse the EMS. Samples (1ml) were removed every 20 minutes and a kill curve determined. After 90 minutes at 25°C the cells were harvested and washed twice in phosphate buffer containing 6%(wt/v) sodium thiosulphate followed by three washes in distilled water. Cells were resuspended in LB broth medium for 2 hours and 30°C to allow expression of mutations.

The mutagenised stock was dispensed in 100 ul aliquots onto basal M9 medium agar containing 100 ug $ml^{-1}$ of p-fluorophenylalanine. Plates were incubated at 30°C for 5 days. Control plates of LB agar were also inoculated with serial dilutions of mutagenised stock so as to

determine the concentration of colony forming units and frequency of resistant colonies arising. Colonies arising on M9 analogue plates were picked and transferred to a second selective M9 analogue (PFP) plate. The latter plates were incubated at 30°C for 4 days. The largest colonies were transferred to 50 ml shake flasks containing 10 ml sterile ACA induction medium and incubated at 30°C for 48 hours and 165 rpm shaking. Cells were harvested by centrifugation washed and resuspended 50 mM phosphate buffer pH 7.0 to 50 mg cell dry weight per ml. Reactions contained 0.2 ml cells suspension and 0.2 ml ACA 2% w/v in 100 mM phosphate buffer pH 6.8. Phenylalanine production was monitored. One particular strain isolated in this manner was found to produce more than 9 $gl^{-1}$ L-phenylalanine and was designated PFP 26.

A colony of PFP 26 grown was re-mutagenised using EMS. The second generation mutants were screened as described above, and one mutant, designated AR1, was found to produce more than 11 $gl^{-1}$ L-phenylalanine from 2% ACA. A cell suspension of this mutant was further mutagenised using NTG as follows:

The cell suspension was transferred to a 50 ml screw cap plastic centrifuge tube to which was added a saturated solution of N-methyl-N'-nitro-N-nitrosoguanidine (NTG, Sigma) to one tenth the initial concentration. The tube was shaken gently and incubated at room temperature for 45 minutes. Samples (1 mM) were removed at intervals

and a kill curve determined. Mutagenised cells were washed with sterile water ten times to remove NTG and finally resuspended in 20 ml of LB broth and incubated for 2 hours at 30$^{o}$C to allow mutations to be expressed. Mutants were selected on agar plates containing o-fluorophenylalanine 10 mM. Colonies exhibiting resistance were restreaked on selective agar prior to screening as follows:

The plates were incubated for 4 days and a single colony from each plate inoculated into a flask of medium (ACA-induction) containing: 1% glucose, 0.5% yeast extract, 0.7% $KH_2PO_4$, 0.4% $Na_2HPO_4$, 0.2% $(NH_4)_2SO_4$, 0.1% $MgSO_4.7H_2O$, trace salts ($Ca^{2+}$, $Fe^{3+}$, $Zn^{2+}$) and adjusted to pH 7.0. Acetamidocinnamate was added as inducer at a final concentration of 2 $gl^{-1}$. Cultures were grown for 24 hours at 30$^{o}$C after which cells were removed by centrifugation, washed twice in 50 mM phosphate buffer pH 7.0 and resuspended in the equal volume of the same buffer. To this suspension (usually 10 ml) was added 1 ml reaction mixture containing 2% ACA in 100 mM phosphate pH 7.0. Samples (50 ul) were removed periodically for 24 hours and assayed using paper chromotography and copper nitrate in methanol at $OD_{505}$. Mutants were assessed for activity on the basis of rate of phenylalanine production and total yield achieved.

One mutant was shown to produce more than 17 $gl^{-1}$ L-phenylalanine from 2.5% acetamidocinnamate and was designated OAR1-16. A viable culture of this strain was deposited with ATCC for storage therein under conditions giving permanence to the culture in viable form and has been allotted accession reference number 53529.

Characteristics

The strain designated Corynebacterium OAR1-16 is a phototrophic derivative, produced by mutagenesis, of Corynebacterium strain EVA-5. Accordingly, the mutant strain exhibits most of the characteristics of the patent strain EVA-5. It differs from EVA-5, however, in that growth occurs in the presence of the phenylalanine analogues p-fluro-D, L-phenylalanine or o-fluro-D, L-phenylalanine at concentrations up to 10 mM. In addition, the mutant stain OAR1-16 exhibits an improved ability to produce L-phenylalanine for ACA.

As regards its growth characteristics, the strain herein designated Corynebacterium OAR1-16 grows well on a range of carbon and nitrogen sources, salts and extracts. It can utilize ACA as sole carbon and nitrogen source for growth.

A typical medium contains glucose, sucrose, fructose or glycerol at 1 - 5% w/v; $(NH_4)_2SO_4$, glutamate or asparagine, 0.2-1%; $KH_2PO_4$, 0.2-1%; $K_2HPO_4$, 0.1-0.4%; $Na_2HPO_4$, 0.05-0.2%; $MgSO_4.7H_2O$, 0.01-0.07%; NaCl, 0.1-1%; $CaCl_2.6H_2O$, 0.0001-0.01%; $FeCl_3.7H_2O$, 0.0001-0.0008%; $ZnSO_4.7H_2O$, 0.0001%; $MnCl_2$, $CuSO_4$, $CoCl_2$, in trace amounts; yeast extract, peptone or meat extract, 0.2-2%. Growth is stimulated by addition of various vitamins, particularly thiamine, biotin, pyridoxal, nicotinic acid, pantothenate and p-aminobenzoic acid, 1-45 $ugl^{-1}$. The strain can

therefore be cultivated on a range of conventional laboratory and commerical media.

The characteristics of Strain OAR1-16 may be summarized as follows:

Morphology

Rods, 0.5-1.0 by 1.0-1.5 um, gram positive. Almost coccoid, non motile, pallisade and "V" forms. Non spore forming colonies cream coloured, circular shiny and mucoid. Black colonies formed on potassium tellurite.

Physiology:

| | |
|---|---|
| Nitrate reduction | - |
| Catalase | + |
| Oxidase | - |
| Capsule | + |
| Spore | - |
| Metachromatic granules | + |
| Acid Fast | - |
| Hemolysin | - |
| Starch hydrolysis | - |
| Gelatin hydrolysis | - |
| Urease | - |
| Casein hydrolysis | - |
| Lipolysis | + |

<u>Acid from:</u>

| | |
|---|---|
| Maltose | - |
| Galactose | - |
| Lactose | - |
| Glycerol | - |
| Arabinose | - |
| Fructose | - |
| Rhamnose | - |
| Xylose | - |
| Glucose | - |
| Melibiose | - |
| Sucrose | - |
| Erythrose | - |
| Sorbitol | - |
| | |
| Optimum temperature | 25$^{o}$C |
| Optimum pH | 5-8 |

OAR1-16 cells are constitutive for conversion of acetamidocinnamate to L-phenylalanine, unlike the patent EVA-5 which is inducible. Thus mutant OAR1-16 can be grown in a wide range of media in the absence of ACA inducer and the resultant cells are capable of transforming ACA to L-phenylalanine in high yield., The nature of the constitutivity of OAR1-16 is at the level of the normally

induced ACA permease and deaminase system. Both these enzymes were synthesised by OAR1-16 in the absence of ACA in culture medium. As a result, ACA could be rapidily taken up and deaminated by cells of OAR1-16 grown in a simple glucose/salt medium. A further property of mutant OAR1-16 is the increased expression of the permease and deaminase thus facilitating a more rapid initial conversion of ACA substrate. Furthermore, expression of the phenylalanine dehydrogenase is also partially constitutive and is markedly higher in OAR1-16 than in EVA-5. The phenylalanine dehydrogenase of OAR1-16 is easily detected in cell-free extract, there is increased synthesis of this enzyme and it is not subject to regulation by in vivo proteolytic modification as found with the parent EVA-5.

Futhermore, mutant OAR1-16 is not sensitive to end product feedback inhibition or ACA substrate inhibition as observed with EVA-5. Cells of OAR1-16 are able to produce L-phenylalanine at maximal rates in the presence of ACA 1-5% (w/v) and also in the presence of L-phenylalanine 1-3% (w/v). Mutant OAR1-16 also has significantly higher activity of the deacetylase enzyme catalysing conversion of N-acetyl-L-phenylalanine to L-phenylalanine in high yields.

CLAIMS

1. A process for the enzymatic conversion of acetamidocinnamic acid (ACA) to L-phenylalanine which comprises either bringing ACA into contact with an ACA deaminase and, at the same time or subsequently, with a phenylalanine dehydrogenase and/or with a transaminase, or bringing ACA into contact with an ACA reductase and, at the same time or subsequently, with a deacetylase.

2. A process for biotransformation according to claim 1 of acetamidocinnamate to L-phenylalanine, which comprises treating acetamidocinnamate under enzyme activity promoting conditions, with an enzyme active in the biochemical pathway of acetamidocinnamate-L-phenylalanine transformation, said enzyme being synthesized by a microbial strain of the genus *Alcaligenes*, the genus *Pseudomonas*, the genus *Corynebacterium*, the genus *Brevibacterium*, the genus *Bacillus* or the genus *Micrococcus*, upon cultivating said strain in a growth medium containing acetamidocinnamate as the sole predominant source of carbon and nitrogen.

3. A process according to claim 1 or claim 2 wherein the biotransformation is conducted with an enzyme synthesized by acetamidocinnamate-stimulated growth of a microbial strain selected from the group consisting of *Pseudomonas* EVA-1, *Bacillus* EVA-3, *Alcaligenes* EVA-4, *Corynebacterium* EVA-5 and *Micrococcus* EVA-14.

4. A process according to any one of claims 1 to 3 wherein the biotransformation is conducted with an enzyme synthesized by acetamidocinnamate-stimulated growth of Corynebacterium EVA-5. (ATCC 53208) or a mutant or variant thereof.

5. A process according to claim 4 wherein the biotransformation is conducted in an aqueous medium at pH 4.5-11 and at a temperature of from 18-50$^{o}$C.

6. A process of any one of claims 2 to 5 wherein the biotransformation is conducted in the presence of viable cells of the selected microbial strain during cultivation of said cells on an acetamidocinnamate-containing substrate.

7. A process for the preparation of L-phenylalanine by biotransformation of acetamidocinnamate, which comprises cultivating a microbial strain selected from the group consisting Pseudomonas EVA-1, Bacillus EVA-3, Alcaligenes EVA-4, Corynebacterium EVA-5 and Micrococcus EVA-14 under growth promoting conditions in a growth medium containing acetamidocinnamate, and recovering L-phenylalanine from the growth medium.

8. A process according to claim 7, wherein the microbial strain is Corynebacterium EVA-5 (ATCC 53208) or a mutant or variant thereof.

9. A process according to claim 8 wherein the pH of the reaction medium is from about 4.5 to about 11.

10. A process according to claim 9 wherein the

pH is from 5 to 8.

11. A process according to any one of claims 7 to 10 wherein the temperature of the reaction medium is from about 18°C to about 50°C.

12. A process according to claim 11 wherein the temperature is from 25°C to 37°C.

13. A process according to any one of claims 1 to 6 wherein the transaminase reaction is promoted with pyridoxal phosphate.

14. A process according to any one of claims 1 to 6 or 13 wherein the deaminase converts the ACA to alpha-amino cinnammic acid which degrades via alpha-imino cinnamic acid and alpha-amino phenyl lactic acid to phenyl pyruvic acid and the phenyl pyruvic acid is brought into contact with the dehydrogenase and/or transaminase sufficiently quickly to prevent significant accumulation of phenyl pyruvic acid in the reaction system.

15. A process according to any one of claims 1 to 6, 13 or 14 wherein the desired combination of enzymes are produced by the same microorganism.

16. A process according to claim 15 wherein the microorganism is a deaminase and dehydrogenase and/or transaminase producing strain.

17. A process according to claim 15 or 16 wherein the microorganism is induced in a nutrient medium at a temperature of 25-40°C, at a pH of 4-9 for a period of 10-72 hours with an initial microorganism cell

concentration of 5-40 g/l.

18. A process according to claim 17 wherein ACA and phenylalanine are present in the medium during induction.

19. A process according to claim 17 or 18 wherein, after induction and before production of L-phenylalanine with the ACA substrate, the microorganism is stored in buffer at 0-4$^{o}$C and pH 5-8.

20. A process according to any one of claims 15 to 19 wherein the microorganism is contacted with a medium including ACA.

21. A process according to claim 20 wherein the microorganism is maintained for 10 to 24 hours in a medium at pH 6-7 at 34-37$^{o}$C with an initial microorganism cell concentration of 2-100 g dry weight/l and an initial ACA concentration of 5-40 g/l.

22. A process according to claim 21 wherein the medium includes one or more of added phenylalanine, lactate, pyruvate, citrate, sorbitol, formate or histidine.

23. A process according to claim 18 or 19 wherein the microorganism includes specific constitutive or inducible permease or membrane translocase system to transport substrate into intact cells.

24. A process according to any one of claims 15 to 22 wherein the cell wall of the microorganism is disrupted by methods known *per se* to release the

intracellular enzyme mixture and the resulting enzyme mixture optionally after removing cell debris, is brought into contact with ACA at pH 6-7 at 34-37°C for 10-24 hours.

25. A novel microbiological isolate strain capable of synthesizing enzymes active in the biochemical pathway of conversion of acetamidocinnamate to L-phenylalanine, said strain being Corynebacterium EVA-5 (ATCC 53208) or a mutant or variant thereof.

26. Corynebacterium EVA-5 (ATCC 53208).

**Biotransformation of Acetamidocinnamate to L-Phenylalanine**

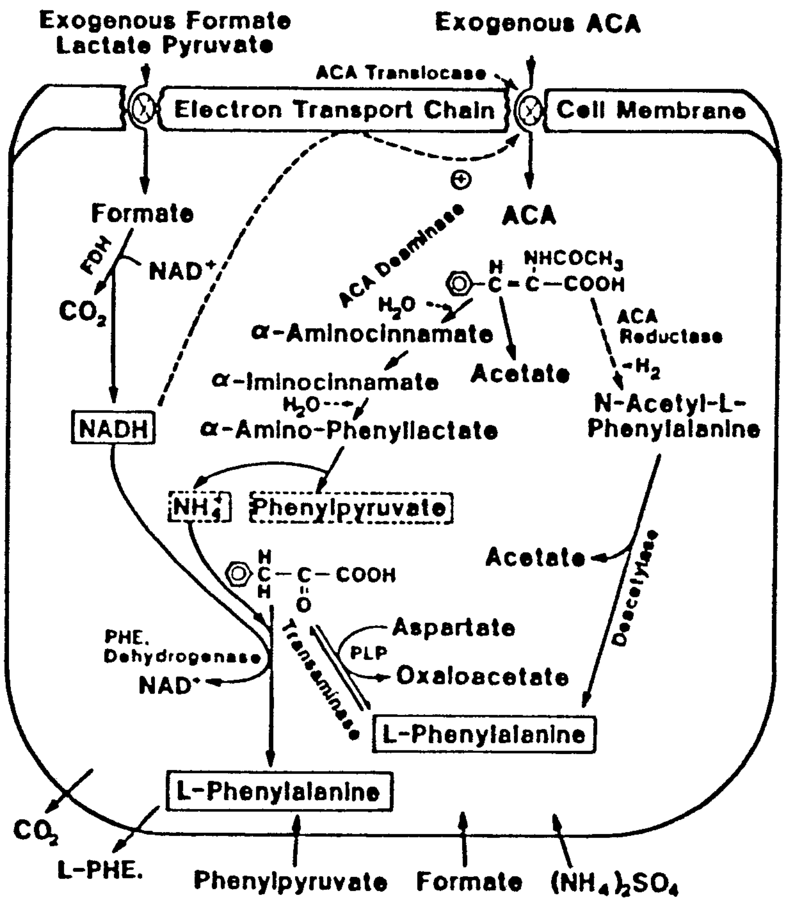


SCHEME 1

FIGURE 1